# EUROPEAN PATENT APPLICATION

(11) **EP 4 442 699 A1**
(43) Date of publication of application: **09.10.2024**
(21) Application number: 23305488.1
(22) Date of filing: 04.04.2023
(51) Int. Cl.: C07K 14/47, C07K 1/22, C07K 14/705

(54) **CRD.VL THERMOSTABLE PROTEIN DOMAINS WITH LECTIN-LIKE PROPERTIES DERIVED FROM GALECTIN 3**

(71) Applicant: Université de Lorraine, 54000 Nancy (FR); Centre National de la Recherche Scientifique, 75016 Paris (FR); Inserm, 75013 Paris (FR)
(72) Inventor: KRIZNIK, Alexandre, 54600 VILLERS-LES-NANCY (FR); QUINTERNET, Marc, 54230 CHALIGNY (FR); REBOUL, Pascal, 54550 SEXEY-AUX-FORGES (FR); BOUTILLIAT, Alexis, 54000 NANCY (FR); CHAGOT, Marie-Eve, 54000 NANCY (FR)
(74) Representative: Novagraaf Technologies

(57) **Abstract**

The present invention concerns new thermostable peptides derived from human galectin-3, in particular from its CRD_{SAT} domain, that are useful to facilitate and improve the purification yield of fusion proteins, in particular of thermostable fusion proteins by simple heating.

## Description

### Technical field of the invention

The present invention concerns new thermostable peptides derived from human galectin-3, in particular from its CRD_{SAT} domain, that are useful to facilitate and improve the purification yield of fusion proteins, in particular of thermostable fusion proteins by simple heating.

The present invention finds its applications mainly in public and private research laboratories, as well as in the pharmaceutical industry having the need to produce recombinant proteins for the purpose of fundamental studies or of therapeutic interest.

In the description below, references in square brackets ([ ]) refer to the list of references at the end of the text.

### State of the art

For several years, the application needs for recombinant proteins have been growing in many economic and research fields, ranging, for example, from the pharmaceutical, chemical and agronomic industries to university laboratories.

A key step for the use of recombinant proteins is their purification from ectopic overexpression systems. For decades, bacterial strains have been optimised to improve the yields, purity, folding and/or safety of proteins encoded on plasmid expression vectors, which are themselves optimised to best perform their task. To facilitate operations, purification tags that can be fused to the protein of interest have been developed. Some tags, such as the SUMO (Small Ubiquitin-like MOdifier) tag, can improve the solubility of the protein construct, but many, including 6xHis (composed of at least six histidines), GST (glutathione S-transferase), MBP (Maltose-Binding Protein), TAP (Tandem Affinity Purification), FLAG and STREP (STREPtavidin), benefit from their ability to bind specifically to agarose or Sepharose resin beads coated with a specific ligand. The affinity for the ligand allows the protein of interest to be retained on the beads, while the proteins in the cell expression system are removed in the washing step. Competition with an eluent molecule, or direct cleavage by a protease specifically recognising a target region embedded in the protein construct, releases the protein of interest from the beads. Thus, the more specific the affinity chromatography step, the purer the eluted fractions.

The thermostability of a recombinant protein can be exploited to increase its purification yield. Indeed, when a protein of interest is overexpressed by bacterial systems such as *E*. *coli,* many endogenous proteins, associated with cell growth, are produced. The thermostability of a recombinant protein can be exploited to increase its purification yield. Indeed, when a protein of interest is overexpressed by bacterial systems such as *E*. *coli,* many endogenous proteins, associated with cell growth, are produced. As these proteins precipitate at temperatures above 55 °C, many of them can be efficiently removed from the soluble bacterial fraction by a simple heating step of a few minutes.

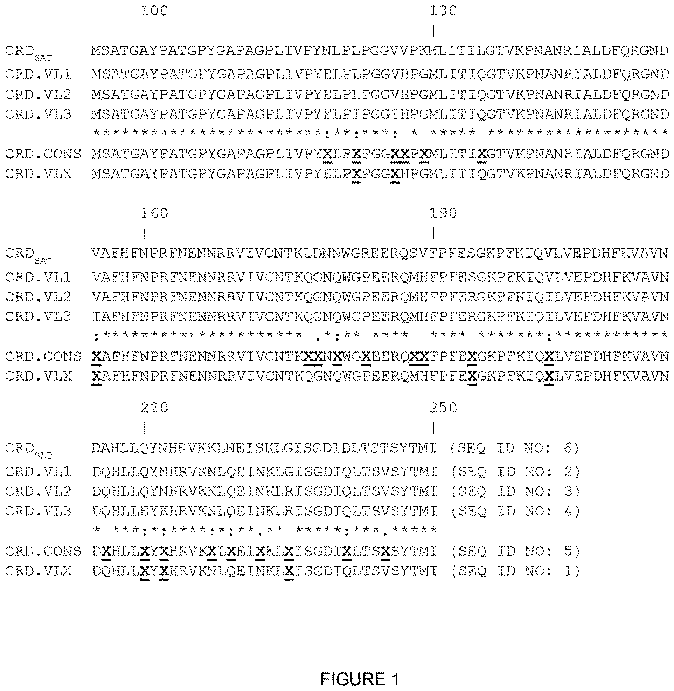

Recently a protein purification tag based on the carbohydrate recognition domain (CRD) of human galectin-3, called CRD_{SAT}, has been developed which allows for improved purification of proteins of interest by affinity chromatography. This protein tag has the advantage of being highly specific for its ligand, of facilitating the solubilisation of the protein to be purified and of working with a low cost system linked to the use of an abundant and inexpensive compound: D-lactose [1]. However, it has limited temperature stability, with a temperature of 55.8 °C measured by differential scanning calorimetry (DSC) (see Figure 1).

### Description of the invention

Molecular engineering can often be used to modify the catalytic, binding or physicochemical properties of proteins. Different approaches are possible, some consisting of random mutagenesis of the peptide sequence, others based on a rationale guided by the three-dimensional structure of the protein. The inventors opted for this second approach to try to improve the stability of the CRD_{SAT}.

The inventors therefore designed variants of CRD_{SAT} with retained lectin activity making it possible to select fusion proteins by affinity chromatography and improved stability at high temperature (up to about 92 °C for instance). These thermostable variants, called CRD.VL protein domains or CRD.VL, when fused to a thermostable protein of interest thus allow to improve the purification yield by simple heating.

An object of the present invention is therefore a CRD.VL protein domain comprising or consisting of a peptide having the following amino acid sequence: 
MSATGAYPATGPYGAPAGPLIVPYELPX28PGGX32HPGMLITIQGTVKPNANRIALDF
QRGNDX61AFHFNPRFNENNRRVIVCNTKQGNQWGPEERQMHFPFEX100GKPFKIQ
X108LVEPDHFKVAVNDQHLLX126YX128HRVKNLQEINKLX141ISGDIQLTSVSYTMI,
 wherein X₂₈, X₃₂, X₆₁, X₁₀₀, X₁₀₈, X₁₂₆, X₁₂₈, and X₁₄₁ are independently of each other any amino acid (SEQ ID NO: 1).

According to a particular embodiment of the CRD.VL protein domain of the invention, X₂₈ is Leu or Ile, X₃₂ is Val or Ile, X₆₁ is Val or Ile, X₁₀₀ is Ser or Arg, X₁₀₈ is Val or Ile, X₁₂₆ is Gln or Glu, X₁₂₈ is Asn or Lys, and/or X₁₄₁ is Gly or Arg (SEQ ID NO: 7).

According to a particular embodiment of the CRD.VL protein domain of the invention, said CRD.VL protein domain comprises or consists of the amino acid sequence:
MSATGAYPATGPYGAPAGPLIVPYELPLPGGVHPGMLITIQGTVKPNANRIALDFQR GNDVAFHFNPRFNENNRRVIVCNTKQGNQWGPEERQMHFPFESGKPFKIQVLVEP DHFKVAVNDQHLLQYNHRVKNLQEINKLGISGDIQLTSVSYTMI (SEQ ID NO: 2) as the CRD.VL1 protein domain,
MSATGAYPATGPYGAPAGPLIVPYELPLPGGVHPGMLITIQGTVKPNANRIALDFQR GNDVAFHFNPRFNENNRRVIVCNTKQGNQWGPEERQMHFPFERGKPFKIQILVEP DHFKVAVNDQHLLQYNHRVKNLQEINKLRISGDIQLTSVSYTMI (SEQ ID NO: 3) as the CRD.VL2 protein domain, or
MSATGAYPATGPYGAPAGPLIVPYELPIPGGIHPGMLITIQGTVKPNANRIALDFQRG NDIAFHFNPRFNENNRRVIVCNTKQGNQWGPEERQMHFPFERGKPFKIQILVEPDH FKVAVNDQHLLEYKHRVKNLQEINKLRISGDIQLTSVSYTMI (SEQ ID NO: 4) as the CRD.VL3 protein domain.

Another object of the present invention is a fusion protein comprising or consisting of a CRD.VL protein domain of the present invention fused to a molecule of interest, with a protease cleavage site inserted between the two.

According to a particular embodiment of the fusion protein of the present invention, the cleavage site is a TEV protease cleavage site.

Another object of the present invention is an plasmidic expression vector comprising a nucleotide sequence encoding a fusion protein of the present invention.

Another object of the present invention is a method for producing a purified protein of interest, said method comprising:
a) preparing an expression vector according to claim 6;
b) transforming a host cell with said expression vector;
c) culturing said transformed host cell under conditions enabling the expression of the fusion protein in the culture medium;
d) optionally heating the culture medium containing the expressed fusion protein at a temperature ranging from 56 to 70 °C, preferably at 60 °C; and
e) purifying the molecule of interest.

According to a particular embodiment of the method of the present invention, the purification step e) is carried out by:
(i) binding the fusion protein via the CRD.VL protein domain onto a chromatography support grafted with lactose molecules, preferably onto a column of agarose or sepharose resin grafted with lactose molecules;
(ii) cleavage by protease of the protein of interest;
(iii) elution of the protein of interest;
(iv) separation of the protease from the protein of interest.

According to a particular embodiment of the method of the present invention, the purification step e) is carried out by:
(i) binding the fusion protein via the CRD.VL protein domain onto a chromatography support grafted with lactose molecules, preferably onto a column of agarose or sepharose resin grafted with lactose molecules;
(ii) elution of the fusion protein;
(iii) cleavage by protease of the protein of interest in solution;
(iv) separation of the protease and the CRD.VL protein domain, from the protein of interest.

According to a particular embodiment of the method of the present invention, the separation step (iv) is carried out by ion exchange chromatography or size exclusion exclusion chromatography or hydrophobic interection chromatography.

According to a particular embodiment of the method of the present invention, the elution step (ii) is carried out with a lactose solution.

### Brief description of the figures

Figure 1 represents protein sequence alignment of CRD.VL1, CRD.VL2 and CRD.VL3 (SEQ ID NOs: 2-4) in view of the protein sequence of CRD_{SAT} (SEQ ID NO: 6). An asterisk indicates positions which have a single, fully conserved residue. A colon indicates conservation between groups of strongly similar properties. A period indicates conservation between groups of weakly similar properties. A first consensus sequence (CRD.CONS, SEQ ID NO: 5) that contains a **X** at positions in the CRD.VL1, CRD.VL2 and CRD.VL3 sequences that do not strictly match with the CRD_{SAT} sequence is reported. A second consensus sequence (CRD.VLX, SEQ ID NO: 1) that contains a **X** at positions in the CRD.VL1, CRD.VL2 and CRD.VL3 sequences that do not strictly match therebetween. The numbering of the sequences is related to the numbering of the full-length wild-type human galectin-3.

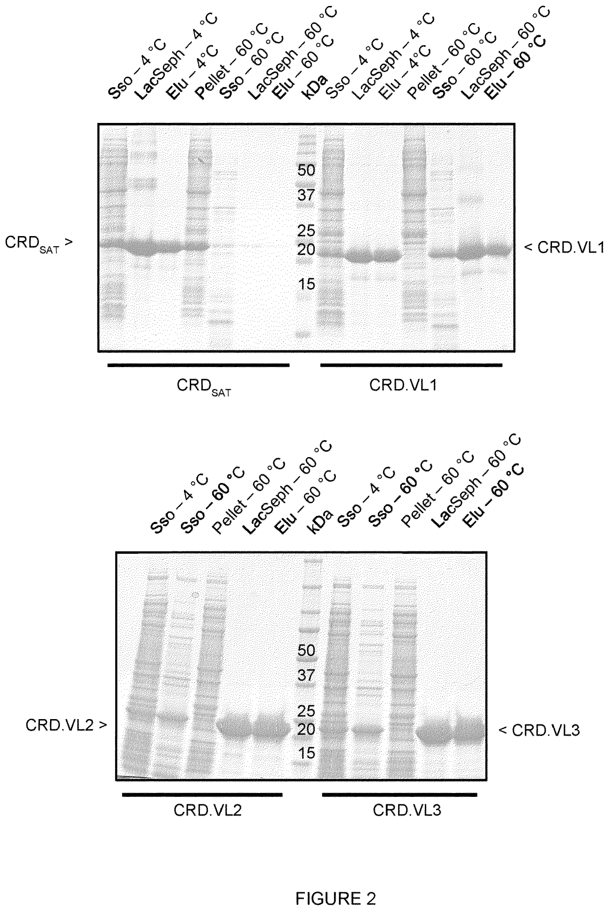

Figure 2 represents SDS-PAGE analysis of the purification of CRD_{SAT}, CD.VL1, CRD.VL2 and CRD.VL3 expressed individually in *E*. *coli* BL21(DE3) pRARE2. « Sso », « LacSeph », « Pellet », « Elu » and « kDa » refer respectively to the supernatant sonicate, the Lactose-Sepharose beads, the Pellet after centrifugation, the elution with D-Lactose and the protein size ladder. The « 4 °C » tag means that the purification step was done at 4 °C. The « 60 °C » tag means that the purification step follows a heating at 60 °C of the sonicated supernatant. Only CRD.VL1, CRD.VL2 and CRD.VL3 are found in the « Elu - 60 °C » tracks and absent from the « Pellet - 60 °C » tracks. CRD_{SAT} is mainly found in the « Elu - 4 °C » and « Pellet - 60 °C » lanes

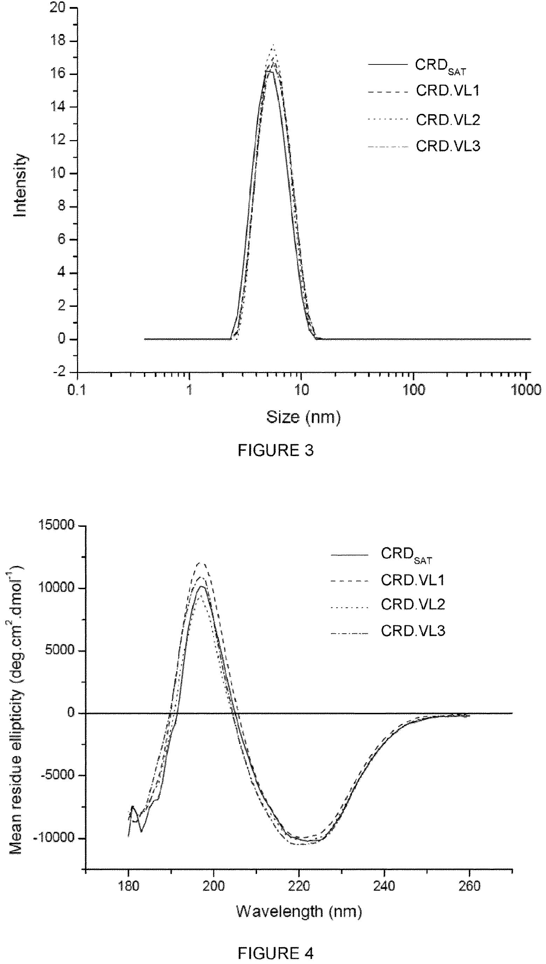

Figure 3 represents dynamic light scattering analysis of CRD_{SAT}, CRD.VL1, CRV.VL2 and CRD.VL3 performed on a Zetasizer apparatus (Malvern, Ltd). Data were recorded at 20 °C in lysis buffer using a protein concentration of 50 µM. The variant proteins size observed with this technique are similar to CRD_{SAT} and is characteristic of a monomeric CRD protein (from 5.2 to 5.9 nm).
Figure 4 represents circular dichroism analysis of CRD_{SAT}, CRD.VL1, CRD.VL2 and CRD.VL3 concentrated at 50 µM in 10 mM NaPi, 100 mM NaF, pH 7.4 buffer, performed on a Chirascan spectrometer (Applied Photophysics, Ltd).

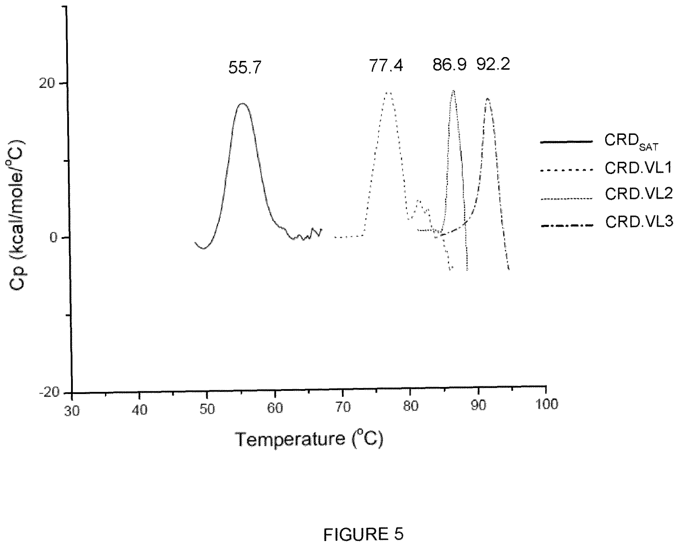

Figure 5 represents differential scanning calorimetry of CRD_{SAT}, CRD.VL1, CRV.VL2 and CRD.VL3 performed on a VP-DSC instrument (MicroCal, Malvern, Ltd) at a 35 µM concentration in 20 mM NaPi, 150 mM NaCl, pH 7.4 buffer. Denaturation curves allowed to measure a mid-point temperature value or T_{M}, corresponding to the to half-denaturation of the protein sample. T_{M} values for CRD_{SAT} equals to 55.7 °C; 77.4 °C for CRD.VL1, 86.9 °C for CRD.VL2 and 92.2 °C for CRD.VL3 respectively. The higher the T_{M} value, the higher the protein thermostability.

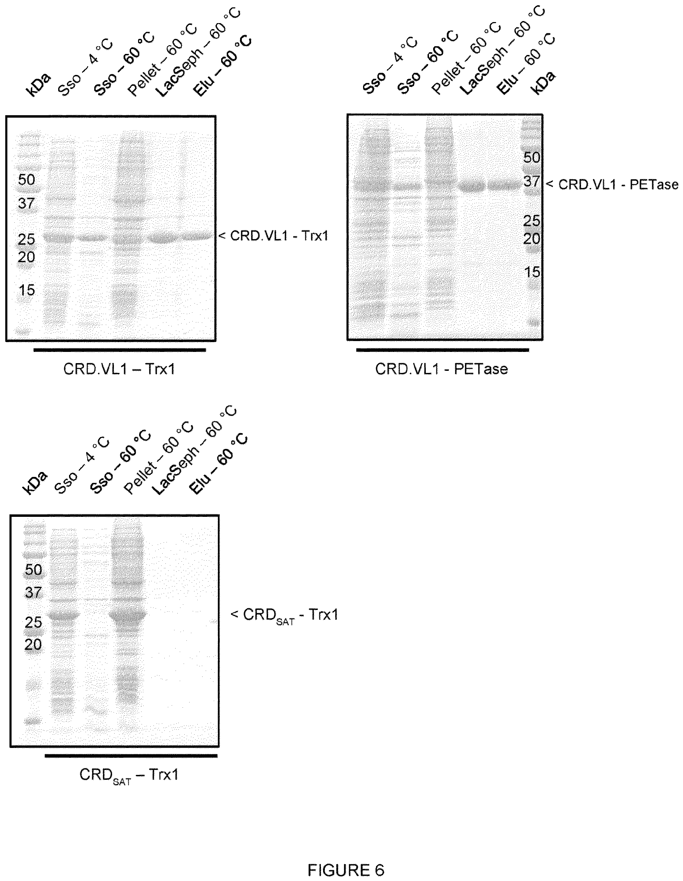

Figure 6 represents SDS-PAGE analysis of the purification of Trx1 and PETase fused to CRD.VL1 and of Trx1 fused to CRD_{SAT} expressed in *E*. *coli* BL21(DE3) pRARE2. « Sso », « LacSeph », « Pellet », « Elu » and « kDa » refer respectively to the sonicated supernatant, the Lactose-Sepharose beads, the pellet after centrifugation, the elution with D-Lactose and the protein size ladder. The « 4 °C » tag means that the purification step was done at 4 °C. The « 60 °C » tag means that the purification step follows a heating at 60 °C of the sonicated supernatant.

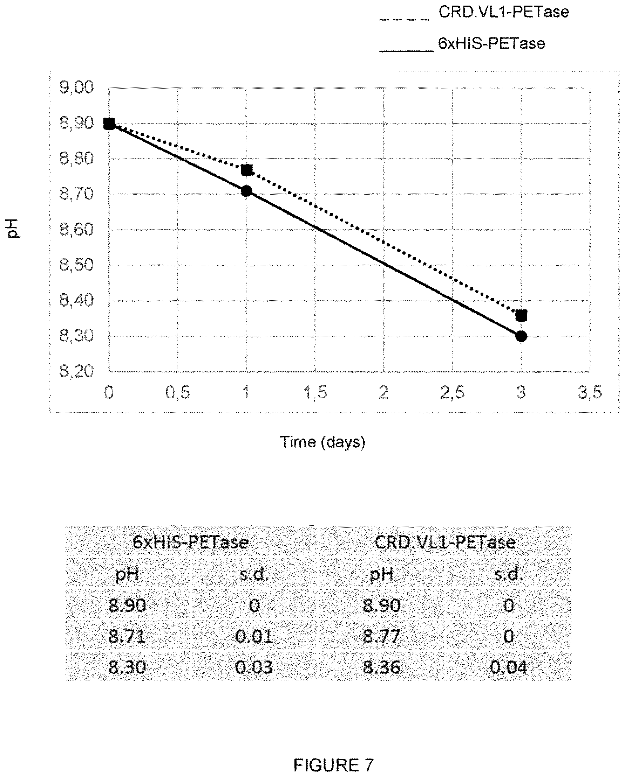

Figure 7 represents PET hydrolysis assay performed at 65 °C with PETase fused to CRD.VL1 or to the 6xHIS tag. Starting pH value of the 100 mM Bicine buffer containing 50 mg of amorphous PET is 8.90.

### EXAMPLES

### EXAMPLE 1 : PRODUCTION OF THERMOSTABLE CRD.VL PROTEIN DOMAINS AND USES THEREOF

### Design of three protein sequences derived from the CRD_{SAT} protein sequence

With the aim to inspect the protein sequence variability of the CRD_{SAT} domain derived from the human Galectin-3 (residue 96 to 250 (plus a Met residue in Nterm) SEQ ID NO: 6), an analysis of its three-dimensional X-ray structure (Kriznik et al., 2019) [2] was undertook using the PROSS webserver (Goldenzweig et al., 2016) [3]. As a result, a maximum of 28 variable position was obtained.

Taken advantage of these positions, three protein sequences were designed, called CRD.VL1, CRD.VL2 and CRD.VL3 (Figure 1; SEQ ID NOs: 2-4). More in details, and in order to preserve the binding properties of the D-lactose, specific to the CRDs, residues in the vicinity of the sugar binding pocket made, in CRD_{SAT}, with residues N160, R162, V172, N174, W181, E184, R186, were not mutated. In addition, residues from the N-terminal tail that contains a polyproline stretch and residues which appear to be important to maintain the overall three-dimensional structure of the protein, were not mutated.

From these variant sequences (SEQ ID NOs: 2-4), a consensus sequence was derived that could be the subject of point mutations, namely: 
MSATGAYPATGPYGAPAGPLIVPYELPX28PGGX32HPGMLITIQGTVKPNANRIALDF
QRGNDX61AFHFNPRFNENNRRVIVCNTKQGNQWGPEERQMHFPFEX100GKPFKIQ
X108LVEPDHFKVAVNDQHLLX126YX128HRVKNLQEINKLX141ISGDIQLTSVSYTMI,
wherein X₂₈, X₃₂, X₆₁, X₁₀₀, X₁₀₈, X₁₂₆, X₁₂₈, and X₁₄₁ are independently of each other any amino acid (SEQ ID NO: 1); or in particular
wherein X₂₈ is Leu or Ile, X₃₂ is Val or Ile, X₆₁ is Val or Ile, X₁₀₀ is Ser or Arg, X₁₀₈ is Val or Ile, X₁₂₆ is Gln or Glu, X₁₂₈ is Asn or Lys, and/or X₁₄₁ is Gly or Arg (SEQ ID NO: 7).

### Purification of recombinant CRD_{SAT}, CRD.VL1, CRD.VL2 and CRD.VL3 using a heating step

To test the isolated overexpression of the three variants described in Figure 1, the sequence of the CRD_{SAT} encoded in the pCARGHO vector [1,2] was modified to match the ones of CRD.VL1, CRD.VL2 and CRD.VL3. A BL21(DE3) pRARE 2 *Escherichia coli* strain was then transformed with a pCARGHO (i.e. without CRD.VL sequence), pCARGHO_{VL1}, pCARGHO_{VL2} or pCARGHO_{VL3} vector as previously shown in [1,2].

For each transformation, a positive clone was selected on LB-agar plates then grown overnight at 37 °C under agitation in liquid LB medium. The latter culture was used to seed a fresh LB medium cultivated at 37 °C under agitation until optical density reached a value of 0.6 at 600 nm. Protein overexpression was induced with addition of 0.25 mM IPTG (Isopropyl β-D-1-thiogalactopyranoside) in the bacterial culture that was then placed at 20 °C under agitation for 16 hours.

Cells were harvested by centrifugation and resuspended in a lysis buffer (25 mM HEPES, 300 mM NaCl, pH 7.5). The suspension was sonicated, then centrifuged in order to discard the insoluble protein fraction. To increase the cleaning rate, the supernatant was heated for 10 minutes at 60 °C to precipitate labile proteins and then, centrifuged. The supernatant resulting from the heating step was mixed for 10 minutes with lactose-Sepharose beads that specifically bind properly folded CRDs. The beads were washed with the lysis buffer before being mixed with the lysis buffer supplemented with 200 mM of D-Lactose to release the protein of interest. This protein purification process was followed by polyacrylamide gel electrophoresis under denaturant conditions (SDS-PAGE).

As a result, it was shown that CRD.VL1, CRD.VL2 and CRD.VL3 expressed individually could support, with no or only weak material loss, a heating step at 60°C in contrary to CRD_{SAT} (Figure 2). Indeed, the latter was entirely found in the pellet resulting from centrifugation that follows the heating step, whereas it remains soluble when the purification is fully performed at 4 °C. It was also noticed that many other soluble proteins coming from the bacterial expression system are also discarded from the supernatant by heat-induced precipitation (See lane Pellet - 60 °C in Figure 2). Importantly, CRD.VL1, CRD.VL2 and CRD.VL3 successfully bind to, then elute from, the lactose-Sepharose. This demonstrates the ability of these variants to specifically bind D-Lactose and strongly suggests that they fold as CRD_{SAT} from which they are derived.

### Structural analysis of CRD.VL1, CRD.VL2 and CRD.VL3

Purified recombinant CRD.VL1, CRD.VL2 and CRD.VL3 were first analyzed using dynamic light scattering (DLS). Data were recorded at 20°C in lysis buffer on a Zetasizer apparatus using a protein concentration of 50 µM. The protein sizes observed with this technique are in the same range than CRD_{SAT} and characteristic of a monomeric CRD protein (Figure 3).

Second, a circular dichroism (CD) analysis of the three variants concentrated at 50 µM in 10 mM sodium phosphate (NaPi), 100 mM NaF, pH 7.4 revealed that spectra of the variants are very close to the one of CRD_{SAT} (Figure 4). Indeed identical curves that overwhelmingly overlap were obtained, which relies on a similar structural conformation between the 3 CRD.VL and the original CRD_{SAT}. The full beta-sheet signature is consistent with the 3D structure of CRD_{SAT} obtained by X-ray method (pdb code : 6H64).

Altogether, it demonstrates, as suggested by their D-lactose binding properties, that CRD.VL1, CRD.VL2 and CRD.VL3 fold as CRD_{SAT}.

### Thermal stability of CRD.VL1, CRD.VL2 and CRD.VL3

As shown in Figure 2, CRD.VL1, CRD.VL2 and CRD.VL3 do not suffer any damage at 60°C since they keep their ability to specifically bind D-lactose after a heating step. To better characterize their thermal stability, which appears undoubtedly greater than the CRD_{SAT} one, differential scanning calorimetry (DSC) was used and recorded on a Microcal VP-DSC in a 0.54 mL cell, at a pressure of 2 atm with a protein concentration of 35 µM in 20 mM NaPi, 150 mM NaCl, pH 7.4. The Tm, corresponding to the midpoint temperature of the unfolding transition, was determined with the Origin 7.0 software (Malvern).

Tm values of 55.7, 77.4, 86.9 and 92.2 °C were measured for CRD_{SAT}, CRD.VL1, CRD.VL2 and CRD.VL3 respectively (Figure 5). This explains the purification data in which CRD_{SAT} precipitated after heating at 60 °C. Indeed, according to the DSC data, this protein domain is unfolded at this temperature and aggregates in an insoluble pellet. In agreement with the DSC results, CRD.VL1, CRD.VL2 and CRD.VL3 remain stable at 60 °C during the purification process and could thus be successfully purified as soluble lectinic domains.

Interestingly, the accumulation of mutations at the positions described in the consensus sequence shown in Figure 1 seems linked to the increase of the thermal stability of the protein domain.

### Using thermostable CRD.VL to purify passenger protein

To take advantage of the thermostability of variants of CRD_{SAT}, we fused CRD.VL1 to a bacterial Thioredoxin (Trx1, Miranda-Vizuete et al., 1997) [4] and to a PET hydrolase (the latter being an enzyme able to hydrolyze polyethylene terephthalate (PET) and turns it into ethylene glycol and terephthalic acid, Tournier et al., 2020) [5]. Both proteins have been shown to be thermostable and active at temperatures above 65 °C.

DNA sequences of the passenger proteins were integrated into a pCARGHO_{VL1} plasmid, downstream a CRD.VL1 tag sequence and a protease TEV cleavage site.

Protein expression and purification were performed as described above, meaning with the inclusion of a heating step at 60 °C performed on the soluble fraction obtained after cell sonication.

In Figure 6, it was shown that this step allowed a very efficient cleaning of the undesired bacterial proteins and did not significantly affect the solubility of the fusion proteins CRD.VL1-Trx1 and CRD.VL1-PETase. Indeed, most of the soluble proteins coming from the *E*. *coli* expression strain were found in the pellet after heating whereas the CRD.VL1-tagged proteins were overwhelmingly kept in the soluble fraction at the end of the purification process, meaning they are found in the « Elu - 60 °C » lane. More importantly, the two CRD.VL1-tagged proteins are successfully selected on and eluted from Lactose-Sepharose beads.

As a control, Trx1 fused to CRD_{SAT} did not withstand the heating step at 60 °C of the Sso since it was found insoluble in the pellet at this stage and therefore could not be selected on Lactose-Sepharose beads (Figure 6).

Finally, we designed a PET hydrolysis assay to test the activity of the CRD.VL1-tagged PETase. A coupon of 50 mg of amorphous PET (Goodfellow) was placed in a 2 mL tube and immerged in 100 mM Bicine buffer, pH 8.9. A final concentration of 500 nM of 6xHIS-PETase or CRD.VL1-PETase, both heat-purified, was introduced in the tube that was next incubated at 65 °C during three days. Hydrolysis of the PET was measured by monitoring the decrease of the pH value of the buffer due to the formation of terephthalic acid upon enzymatic digestion. This assay was performed two times to estimate the reproducibility of the results.

A decrease in the pH value was observed after 1 and 3 days due to the release of terephtalic acid upon PET hydrolysis (Figure 7). Mean and standard-deviation of the pH value were reported on the graphic as well as in the table below. With similar pH values obtained after 3 days, therefore it was shown that the heat-purified PETase fused to CRD.VL1 possessed an enzymatic activity comparable to the his-tagged PETase (Figure 7).

### Conclusion

In conclusion, substitutable positions in the protein sequence of CRD_{SAT} were identified, as well as associated amino acids to be favored, to increase the thermal stability of this domain derived from the human galectin-3 (Figure 1). The substitute amino acids to be preferred will be those found in CRD.VL3 because the latter presents the greatest Tm value.

Monomeric and thermostable variants of the CRD_{SAT}, such as CRD.VL1, CRD.VL2 and CRD.VL3, could be effectively used as lectinic purification tags without altering the potential enzymatic activity of a passenger protein (protein of interest). They can simplify, speed up and improve the purification of the passenger protein using a heating step followed by D-Lactose affinity chromatography. The low cost of this process may be advantageous for large scale exploitation of these protein tags.

### List of references

[1] International Application WO 2017/194888
[2] Kriznik et al., Biotechnol. J., 14(4) : e1800214, 2019; doi :10.1002/biot.201800214
[3] Goldenzweig, A. et al., Mol. Cell, 63(2): 337-346, 2016
[4] Miranda-Vizuete et al., J. Biol. Chem., 272(49) : 30841-30847, 1997
[5] Tournier et al., Nature, 580(7802) : 216-219, 2020

## Claims

1. CRD.VL protein domain comprising a peptide having the following amino acid sequence: 
MSATGAYPATGPYGAPAGPLIVPYELPX28PGGX32HPGMLITIQGTVKPNANRIALDF
QRGNDX61AFHFNPRFNENNRRVIVCNTKQGNQWGPEERQMHFPFEX100GKPFKIQ
X108LVEPDHFKVAVNDQHLLX126YX128HRVKNLQEINKLX141ISGDIQLTSVSYTMI,
 wherein X₂₈, X₃₂, X₆₁, X₁₀₀, X₁₀₈, X₁₂₆, X₁₂₈, and X₁₄₁ are independently of each other any amino acid (SEQ ID NO: 1).

2. CRD.VL protein domain according to claim 1, wherein X₂₈ is Leu or Ile, X₃₂ is Val or Ile, X₆₁ is Val or Ile, X₁₀₀ is Ser or Arg, X₁₀₈ is Val or Ile, X₁₂₆ is Gln or Glu, X₁₂₈ is Asn or Lys, and/or X₁₄₁ is Gly or Arg (SEQ ID NO: 7).

3. CRD.VL protein domain according to claim 1 or 2 comprising the amino acid sequence/

MSATGAYPATGPYGAPAGPLIVPYELPLPGGVHPGMLITIQGTVKPNANRIALDFQR
GNDVAFHFNPRFNENNRRVIVCNTKQGNQWGPEERQMHFPFESGKPFKIQVLVEP
DHFKVAVNDQHLLQYNHRVKNLQEINKLGISGDIQLTSVSYTMI (SEQ ID NO: 2),
MSATGAYPATGPYGAPAGPLIVPYELPLPGGVHPGMLITIQGTVKPNANRIALDFQR
GNDVAFHFNPRFNENNRRVIVCNTKQGNQWGPEERQMHFPFERGKPFKIQILVEP
DHFKVAVNDQHLLQYNHRVKNLQEINKLRISGDIQLTSVSYTMI (SEQ ID NO: 3), or
MSATGAYPATGPYGAPAGPLIVPYELPIPGGIHPGMLITIQGTVKPNANRIALDFQRG
NDIAFHFNPRFNENNRRVIVCNTKQGNQWGPEERQMHFPFERGKPFKIQILVEPDH
FKVAVNDQHLLEYKHRVKNLQEINKLRISGDIQLTSVSYTMI (SEQ ID NO: 4).

4. Fusion protein comprising a CRD.VL protein domain as defined in any of claims 1 to 3 fused to a molecule of interest, with a protease cleavage site inserted between the two.

5. Fusion protein according to claim 4, wherein the cleavage site is a TEV protease cleavage site.

6. Expression vector comprising a nucleotide sequence encoding a fusion protein as defined in any of claims 4 or 5.

7. Method for producing a purified protein of interest, said method comprising:
a) preparing an expression vector according to claim 6;
b) transforming a host cell with said expression vector;
c) culturing said transformed host cell under conditions enabling the expression of the fusion protein in the culture medium;
d) optionally heating the culture medium containing the expressed fusion protein at a temperature ranging from 56 to 70 °C, preferably at 60 °C; and
e) purifying the molecule of interest.

8. Method according to claim 7, wherein the purification step e) is carried out by:
(i) binding the fusion protein via the CRD.VL protein domain onto a chromatography support grafted with lactose molecules, preferably onto a column of agarose or sepharose resin grafted with lactose molecules;
(ii) cleavage by protease of the protein of interest;
(iii) elution of the protein of interest;
(iv) separation of the protease from the protein of interest.

9. Method according to claim 7, wherein the purification step e) is carried out by:
(i) binding the fusion protein via the CRD.VL protein domain onto a chromatography support grafted with lactose molecules, preferably onto a column of agarose or sepharose resin grafted with lactose molecules;
(ii) elution of the fusion protein;
(iii) cleavage by protease of the protein of interest in solution;
(iv) separation of the protease and the CRD.VL protein domain, from the protein of interest.

10. Method according to any one of claims 8 or 9, wherein the separation step (iv) is carried out by ion exchange chromatography or size exclusion exclusion chromatography or hydrophobic interection chromatography.

11. Method according to any one of claims 8 to 10, wherein the elution step (ii) is carried out with a lactose solution.
